# EUROPEAN PATENT APPLICATION

(11) **EP 3 769 767 A1**
(43) Date of publication of application: **27.01.2021**
(21) Application number: 19188477.4
(22) Date of filing: 25.07.2019
(51) Int. Cl.: A61K 31/7028, A61P 3/10, A61P 13/12

(54) **USE OF SOTAGLIFLOZIN FOR THE TREATMENT OF PATIENTS WITH TYPE 2 DIABETES MELLITUS AND MODERATE RENAL IMPAIRMENT**

(71) Applicant: Lexicon Pharmaceuticals, Inc., The Woodlands, TX 77381 (US)
(72) Inventor: The designation of the inventor has not yet been filed
(74) Representative: Abel & Imray

(57) **Abstract**

The invention relates to the use of sotagliflozin in patients with type 2 diabetes mellitus and moderate renal impairment.

## Description

The invention relates to the use of sotagliflozin for the treatment of patients with type 2 diabetes mellitus and moderate renal impairment.

Described in document WO 2008/10959, sotagliflozin is a dual inhibitor of the sodium-glucose cotransporters type 1 and 2 (SGLT1 and SGLT2).

Sotagliflozin has the following formula:

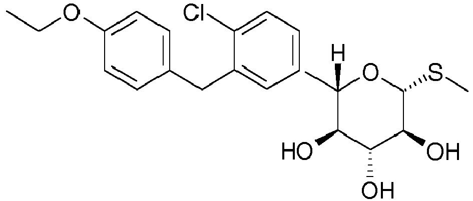

Sotagliflozin is being developed for use in Type 2 diabetes (T2D or type 2 diabetes mellitus), a metabolic disorder characterized by hyperglycemia that results from a combination of increased insulin resistance and beta-cell dysfunction.

According to the most recent International Diabetes Federation Diabetes Atlas, the estimates in 2015 were that 1 in 11 adults have diabetes, which means 415 million people and estimated to be 642 million by 2040.

According to the World Health Organization, there are about 60 million people with diabetes in the European Region, or about 10.3% of men and 9.6% of women aged 25 years and over.

While these numbers include both people with T2D and Type 1 diabetes (T1D), over 90% of adults with diabetes have T2D. Diabetes is among the leading causes of death by disease and is a leading cause of heart disease, stroke, blindness, kidney disease, and amputation. Despite the fact that the population of people with diabetes is growing, none of the current therapies is curative and the results of treatment are variable.

In particular, in spite of the numerous treatment options available, monotherapy fails in many patients as beta-cell function continues to deteriorate leading to progressively increasing hyperglycemia.

Glycemic control with the currently available agents often leads to side effects, most notably weight gain and an increased frequency of hypoglycemia. These concerns emphasize the need to develop new agents that effectively and safely control glucose levels in diabetic patients.

Although blood glucose measurement is essential for assessing glycemic control, there are diurnal variations in blood glucose levels. An indicator of long-term glycemic control is necessary. Glycated haemoglobin (HbA1 c or A1C) HbA1c is the gold standard measurement for the assessment of glycemic control as it reflects average plasma glucose over the previous eight to 12 weeks (Rinsho Byori. 2014 Jan;62(1):45-52.).

Often diabetes is not only related with poo glycemic control but is also associated with various complications. In particular, micro vascular complications of diabetes are well known and can result in impaired renal function, retinopathy and neuropathy and the macrovascular complications result in coronary disease, amputations and stroke..

In particular, diabetic nephropathy is a well-established complication of poor glycemic control in patients with diabetes. In particular, with advancing duration of diabetes and the treatment of T2D in the face of advancing renal insufficiency, diabetic nephropathy represents a particular challenge to the health care providers. An estimated 10-36% of patients with T2DM have some degree of renal impairment and chronic kidney disease (CKD) is present in approximately 40% of patients with diabetes.

CKD is classified into 5 stages, depending on the eGFR (estimated glomerular filtration rate) levels:
- stage 1 corresponds to kidney damage with normal kidney function with a GFR (mL/min/1.73 m2) of 90 or higher;
- stage 2 corresponds to kidney damage with a mild decrease of kidney function and a GFR from 60 to 89;
- stage 3 corresponds to a moderate decrease of kidney function with a GFR from 30 to 59; within stage 3, two stages are differentiated:
   ∘ stage 3a corresponds to a mild to moderate decrease of kidney function with a GFR from 45 to 60, and
   ∘ stage 3b corresponds to a moderate to severe loss of kidney function with a GFR from 40 to 45;
- stage 4 corresponds to a severe decrease in GFR (GFR 15-29); and
- stage 5 corresponds to kidney failure with a GFR lower than 15.

The use of a number of anti-diabetes agents is restricted in patients with renal impairment as many agents used to treat T2D, including sulfonylurea, metformin and incretin-based insulin secretagogues, can no longer be used or must be dosed with caution in the clinical setting of renal insufficiency.

Therefore, there is an unmet need of diabetes treatments improving glycemic control in patients with renal impaired patients.

During phase II trials, sotagliflozin has demonstrated an ability to significantly improve glycemic control in patients with type 2 diabetes, in particular by achieving a reduction in A1 C (Lapuerta et al., Diabetes & Vascular Disease Research2015, Vol. 12(2) 101-110).

It has now been demonstrated that sotagliflozin improves glycaemic control in adults with type 2 diabetes mellitus and renal impairment, in particular moderate renal impairment.

A subject matter described hereinafter thus relates to the use of sotagliflozin, in particular a dose of 400mg of sotagliflozin, to improve glycaemic control in patients with type 2 diabetes mellitus and renal impairment, in particular moderate renal impairment.

Especially, the subject matter relates to the use of sotagliflozin, in particular a dose of 400mg of sotagliflozin, in patients with type 2 diabetes mellitus and CKD3, in particular CKD3a.

In particular, a subject matter described here is the use of sotagliflozin, in particular a dose of 400mg of sotagliflozin, wherein said patients have failed to achieve adequate glycaemic control.

In particular, a subject matter described here is the use of a dose of 400mg of sotagliflozin, wherein said patients have inadequate glycemic control.

Another subject matter described here is the use of a dose of 400mg of sotagliflozin, as an adjunct to insulin or metformin therapy to improve glycaemic control in adults with type 2 diabetes mellitus and CKD3, in particular CKD3a.

In an aspect said patients have failed to achieve adequate glycaemic control despite optimal insulin therapy.

Another subject matter described here is the use of a dose of 400mg of sotagliflozin, wherein sotagliflozin is administered once daily.

Another subject matter described here is the use of a dose of 400mg of sotagliflozin, wherein sotagliflozin is administered before the first meal of the day.

Another subject matter described here is a method of treating type 2 diabetes comprising administering to a patient in need thereof a daily dose of 400mg of sotagliflozin, wherein said patient has type 2 diabetes and renal impairment, in particular CKD3, more particularly CKD3a.

Another subject matter described here is a method of improving glycemic control comprising administering to a patient in need thereof a daily dose of 400mg of sotaglilfozin, wherein said patient has type 2 diabetes and renal impairment, in particular CKD3, more particularly CKD3a.

Another subject matter described here is a method of improving A1C comprising administering to a patient in need thereof an effective a daily dose of 400mg of sotagliflozin, wherein said patient has type 2 diabetes and renal impairment, in particular CKD3, more particularly CKD3a.

In an aspect, sotagliflozin is administered as an adjunct to insulin or metformin therapy.

Another subject matter described here is a method according to anyone wherein sotagliflozin administration is initiated in a patient who had failed to achieve adequate glycaemic control despite optimal insulin therapy.

Another subject matter described here is a dose of 400mg of sotagliflozin for the treatment of type 2 diabetes in patients with renal impairment, in particular CKD3, more particularly CKD3a.

Another subject matter described here is a dose of 400mg of sotagliflozin for the treatment to improve glycaemic control in patients with type 2 diabetes renal impairment, in particular CKD3, more particularly CKD3a.

In another aspect, the dosage of 400 mg of sotagliflozin is administered as twice a 200mg tablet.

### Example 1:

The efficacy, relative to a placebo, of sotagliflozin as adjunct therapy in adult patients with Type 1 Diabetes Mellitus who have inadequate glycemic control with insulin therapy, was evaluated in multicentre, double-blind clinical with random distribution in three groups of treatment (group treated with sotagliflozin 200 mg daily dose, group treated with sotagliflozin 400 mg daily dose, and group treated with a placebo) of patients.

This study was conducted in North America.

### I. Patient selection

The selected patients were adult patients with T2D (drug-naïve or on antidiabetic therapy) and documented moderate renal insufficiency defined by an eGFR (based on the 4 variable Modification of Diet in Renal Disease [MDRD] equation) of ≥30 and <60 mL/min/1.73 m² (CKD 3A, 3B).
Patients continued treatment with insulin(s) or insulin analog(s) during the studies.

The patient recruitment was carried out by taking into account the following inclusion criteria:

### Inclusion criteria

- Patients with T2D (drug-naïve or on antidiabetic therapy) and documented moderate renal insufficiency defined by an eGFR (based on the 4 variable MDRD equation) of ≥30 and <60 mL/min/1.73 m² (CKD 3A, 3B).
- Patient has given written informed consent to participate in the study in accordance with local regulations..

### Exclusion criteria:

1) Exclusion criteria related to study methodology
   - At the time of Screening age <18 years or < legal age of majority, whichever is greater.
   - Body Mass Index (BMI) ≤20 or >45 kg/m2 at Screening.
   - Use of systemic glucocorticoids (excluding topical, intra-articular, or ophthalmic application, nasal spray, or inhaled forms) for more than 10 consecutive days within 90 days prior to the Screening Visit.
   - Use of weight loss medications within 12 weeks or weight change of 5 kg or more during the 12 weeks before Screening.
   - Likelihood of requiring treatment during the study period with drugs not permitted by the study protocol (eg, long-term systemic glucocorticoids) and refusing or unable to take alternative treatment.
   - Patients who have previously participated in any clinical trial of sotagliflozin
   - Patients with severe anemia, severe CV (including congestive heart failure New York Heart Association IV), respiratory, hepatic, neurological, psychiatric, or active malignant
   - tumor, or other major systemic disease or patients with short life expectancy that, according to the Investigator, make implementation of the protocol or interpretation of the study results difficult.
   - Current diagnosis of chronic hepatitis, Hepatitis B surface antigen positive, Hepatitis C antibody positive, and/or other clinically active liver disease requiring treatment.
   - Known presence of factors that interfere with the Central Lab HbA1c measurement (eg, genetic Hb variants) compromising the reliability of HbA1c assessment or medical conditions that affect interpretation of HbA1c results (eg, blood transfusion or severe blood loss in the last 3 months prior to randomization, any condition that shortens erythrocyte survival).
   - History of drug or alcohol abuse within 6 months prior to Screening.
   - Patient is an employee of the Sponsor, or is the Investigator or any Sub-investigator, research assistant, pharmacist, study coordinator, other staff or relative thereof directly involved in conducting the study.
   - Patient who has taken other investigational drugs or prohibited therapy for this study within 12 weeks or 5 half-lives from prior to Screening, whichever is longer.
2) Exclusion criteria related to the diabetes or CKD history and treatment
   - Type 1 diabetes mellitus.
   - HbA1c <7% or HbA1c >11% measured by the central laboratory at Screening.
   - Oral antidiabetic agent or insulin use if dose not stable for 8 weeks before randomization
   - (ie, oral agents changed during past 8 weeks or total daily insulin dose increased or decreased by more than 20% during the past 4 weeks).
   - Use of a selective SGLT2 inhibitor (eg, canagliflozin, dapagliflozin, or empagliflozin) within 12 months prior to the trial.
   - History of DKA or nonketotic hyperosmolar coma within 12 weeks prior to the Screening Visit.
   - History of severe hypoglycemia that resulted in unconsciousness, coma or hospitalization within 6 months prior to the Screening Visit.
   - Renal disease that required treatment with immunosuppressive therapy within the last 12 months, or a history of dialysis or renal transplant or initiation of chronic dialysis within 4 weeks prior to the Screening Visit or expected to occur during the study duration.
   - History of hereditary glucose galactose malabsorption or primary renal glucosuria.
   - Chronic dialysis or CKD stage 4 or 5 or requiring chronic dialysis within 6 months of Screening.
   - Reversible causes of renal failure such as obstruction within 6 months of Screening.
3) Exclusion criteria related to the current knowledge of sotagliflozin
   - Pregnant (confirmed by serum pregnancy test at Screening) or breastfeeding women.
   - Women of childbearing potential not willing to use highly effective method(s) of birth control or who are unwilling or unable to be tested for pregnancy (see Appendix A), during the study.
   - Mean of 3 separate BP measurements >180 mmHg (SBP) or >100 mmHg (DBP) (with or without hypertensive medications).
   - Systolic BP <120 mmHg or DBP <60 mmHg if the patient is on antihypertensive medications.
   - History of hypertensive emergency within 12 weeks prior to Screening.
   - History of gastric surgery including history of gastric banding or inflammatory bowel disease within 3 years prior to the Screening Visit.
   - Difficulty swallowing such that the patient cannot take the Investigational Medical Product (IMP).
   - Known allergies, hypersensitivity, or intolerance to SGLT2 inhibitor or any inactive component of sotagliflozin or placebo (ie, microcrystalline cellulose, croscarmellose sodium [disintegrant], talc, silicone dioxide, and magnesium stearate [nonbovine]), unless the reaction is deemed irrelevant to the study by the Investigator.
   - Laboratory findings with the central lab tests at Visit 1.
      ∘ ALT or aspartate aminotransferase (AST) >3 times the ULN
      ∘ Total bilirubin >1.5 times the ULN (except in case of Gilbert's syndrome)
      ∘ Neutrophils <1500/mm3 (or according to ethnic group) and/or platelets <100 000/mm3
      ∘ Amylase and/or lipase >3 times the ULN.
   - Any country-related specific regulation that would prevent the patient from entering the study.
4) Additional exclusion criteria during or at the end of the Run-in phase before Randomization
   - Patients unwilling or unable to perform self-monitoring of blood glucose (SMBG), complete the patient diary or comply with study visits and other study procedures as required per protocol.
   - Patient insufficiently compliant during Run-in phase based upon tablet count (<80%) or in the opinion of the Investigator.
   - Informed consent withdrawal before randomization (patient who is not willing to continue or fails to return).
   - Any clinically significant abnormality identified on physical examination, laboratory tests, electrocardiogram (ECG) or vital signs at the time of screening or any AE during screening period which, in the judgment of the Investigator or any Sub-investigator, would preclude safe completion of the study or constrains efficacy assessment.

### II. Duration and treatment

The study was conducted as follows:
- Screening Phase -Visit 1(up to 2 weeks)
- Run-in phase (Visit 2), lasted 2 weeks. Patients were treated in a single-blind manner with two placebo tablets (identical to sotagliflozin 200 mg in appearance) administered once daily during the Run-in phase, starting from Visit 2
- Double-blind Treatment Period (Day 1 to Week 26): Eligible patients will be randomized on Day 1 (Visit 3). Following randomization, patients will be treated in a double-blind manner for 52 weeks. A total of 787 patients ≥18 years of age (or ≥ legal age of the majority, whichever is greater) were randomly assigned 1:1:1 to the following 3 treatment groups:
   ∘ Placebo, given as two (2) placebo tablets (identical to sotagliflozin 200 mg in appearance), once daily, before the first meal of the day
   ∘ Sotagliflozin 200 mg, given as 2 tablets: 1 sotagliflozin 200 mg tablet and 1 placebo tablet (identical to sotagliflozin 200 mg in appearance), once daily
   ∘ Sotagliflozin 400 mg, given as two (2) 200 mg tablets, once daily, before the first meal of the day.
- Double-blind Extension Period (Week 27 to Week 52):
- Follow-up Period Following the last dose of the IMP: a 4 week post-treatment visit to collect safety and some efficacy information.

The primary endpoint of this study was to demonstrate the superiority of sotagliflozin 400 mg and 200 mg versus placebo with respect to hemoglobin A1c (HbA1c) reduction at Week 26 in patients with Type 2 diabetes (T2D) who have inadequate glycemic control and moderate renal impairment.

### III. Results

Efficacy analysis was based on the intent-to-treat (ITT) population using efficacy assessments obtained during the study, including those obtained after investigational medicinal product (IMP) discontinuation or introduction of rescue therapy. The ITT population consisted of all randomized patients.
The primary efficacy endpoint was analyzed using an analysis of covariance (ANCOVA) model. The ANCOVA model included treatment groups (sotagliflozin 200 mg, sotagliflozin 400 mg, placebo), randomization stratum of HbA1c (≤8.5%, >8.5%), randomization stratum of SBP (<130, ≥130 mmHg), randomization stratum of CKD stage (3A, 3B), and country as fixed effects, and baseline HbA1c value as a covariate.

Table 1 shows that sotagliflozin achieved its primary endpoint by leading to statistically significant lower A1C levels for the 400 mg dose when compared to placebo after the core treatment period.

| HbA1c (%) | Placebo (N=260) | Sotagliflozin 200 mg (N=263) | Sotagliflozin 400 mg (N=264) |
|---|---|---|---|
| Baseline | | | |
| Number | 260 | 262 | 264 |
| Mean (SD) | 8.18 (1.06) | 8.17 (0.91) | 8.20 (0.94) |
| Median | 8.00 | 8.10 | 8.10 |
| Min: Max | 6.0 : 11.8 | 6.0: 11.4 | 6.6 : 11.0 |
| | | | |

| Week 26 | | | |
|---|---|---|---|
| Number | 233 | 250 | 238 |
| Mean (SD) | 7.90 (1.04) | 7.82 (0.99) | 7.72 (1.00) |
| Median | 7.80 | 7.70 | 7.60 |
| Min: Max | 5.7 : 12.5 | 5.8: 11.4 | 5.2 : 12.9 |
| | | | |

| Change from baseline to Week 26 | | | |
|---|---|---|---|
| Number | 260 | 263 | 264 |
| LS Mean (SE) ^{a,b} | -0.22 (0.061) | -0.32 (0.060) | -0.46 (0.060) |
| | | | |
| LS Mean difference (SE) vs. placebo ^{a,b} | | -0.09 (0.076) | -0.23 (0.076) |
| 95% CI | | (-0.244, 0.054) | (-0.382, -0.082) |
| p-value | | 0.2129 | 0.0024 |

| | | | |
|---|---|---|---|
| CI: Confidence interval; CKD: Chronic Kidney Disease; HbA1c: Hemoglobin Alc; LS: Least squares; SBP: Systolic Blood Pressure; SD: Standard deviation; SE: Standard error of the mean. ^{a} Missing data are imputed using control-based copy reference multiple imputation under the missing not at random framework. Multiple datasets are generated with each containing complete (i.e., non-missing) data. For each complete dataset, the change from baseline to Week 26 is analyzed using ANCOVA model. Results from each complete dataset are combined using Rubin's rule. ^{b} Analysis of covariance (ANCOVA) model includes treatment groups (sotagliflozin 400 mg, sotagliflozin 200 mg, and placebo), randomization strata of HbA1c (≤ 8.5%, > 8.5%) at screening, randomization strata of mean SBP (< 130 mmHg, ≥ 130 mmHg) at screening, randomization stratum of CKD stage (3A, 3B) at screening, and country as fixed effects, and baseline HbA1c as a covariate. PGM=PRODOPS/SAR439954/EFC14837/CIR_EX_01/REPORT/PGM/eff_pchghba1c_wk26_imp_i_t.sas OUT=REPORT/OUTPUT/eff_pchghbalc_wk26_imp_i_t_i.rtf (24JUN2019 - 7:11) | | | |

Comparing sotagliflozin 400 mg group versus placebo in HbA1c:
∘ For overall population, the least squared (LS) mean changes in HbA1c from baseline to W26 were -0.46% and -0.22% in sotagliflozin 400 mg and placebo, reaching the mean HbA1c levels of 7.72% and 7.90% respectively. The LS mean difference were statistically significant (LS mean differences versus placebo were - 0.23 %; 95% CI: -0.382% to -0.082%; p=0.0024)
∘ For CKD3A population, the least squared (LS) mean changes in HbA1c from baseline to W26 were -0.54% and -0.23% in sotagliflozin 400 mg and placebo, reaching the mean HbA1c levels of 7.62% and 7.89% respectively. The LS mean difference were statistically significant (LS mean differences versus placebo were - 0.31 %; 95% Cl: -0.522% to -0.100%; p=0.0039)
∘ For CKD3B population, the least squared (LS) mean changes in HbA1c from baseline to W26 were -0.37% and -0.22% in sotagliflozin 400 mg and placebo, reaching the mean HbA1c levels of 7.82% and 7.90% respectively. The LS mean difference were not statistically significant (LS mean differences versus placebo were - 0.15%; 95% Cl: -0.366% to 0.059% ; p=0.1577).

These results show that sotagliflozin, especially at a daily dose of 400mg improved glycemic control in renal impaired patients, in particular patients with CKD3, and more particularly patients with CKD3a.

## Claims

1. Use of a dose of 400mg of sotagliflozin to improve glycaemic control in patients with type 2 diabetes mellitus and moderate renal impairment.

2. Use of a dose of 400mg of sotagliflozin according to claim 1 in patients with type 2 diabetes mellitus and CKD3.

3. Use of a dose of 400mg of sotagliflozin according to claim 1 or 2 in patients with type 2 diabetes mellitus and CKD3a.

4. Use of a dose of 400mg of sotagliflozin according to anyone of claim 1 to 3 wherein said patients have failed to achieve adequate glycaemic control.

5. Use of a dose of 400mg of sotagliflozin according to anyone of claim 1 to 4, as an adjunct to insulin or metformin therapy to improve glycaemic control in adults with type 2 diabetes mellitus and CKD3.

6. Use of a dose of 400mg of sotagliflozin according to anyone of claim 1 to 5, as an adjunct to insulin or metformin therapy to improve glycaemic control in adults with type 2 diabetes mellitus and CKD3a.

7. Use of a dose of 400mg of sotagliflozin according to claim 5 or 6, wherein said patients have failed to achieve adequate glycaemic control despite optimal insulin therapy.

8. Use of a dose of 400mg of sotagliflozin according to anyone of claims 1 to 7, wherein sotagliflozin is administered once daily.

9. Use of a dose of 400mg of sotagliflozin according to anyone of claims 1 to 8, wherein sotagliflozin is administered before the first meal of the day.

10. Method of treating type 2 diabetes comprising administering to a patient in need thereof a daily dose of 400mg of sotagliflozin, wherein said patient has type 2 diabetes and CKD3.

11. Method of improving glycemic control comprising administering to a patient in need thereof a daily dose of 400mg of sotaglilfozin, wherein said patient has type 2 diabetes and CKD3.

12. Method of improving A1C comprising administering to a patient in need thereof an effective a daily dose of 400mg of sotagliflozin, wherein said patient has type 2 diabetes and CKD3.

13. Method according to anyone of claims 10 to 12 wherein sotagliflozin is administered as an adjunct to insulin or metformin therapy.

14. Method according to anyone of claims 10 to 13, wherein sotagliflozin administration is initiated in a patient who had failed to achieve adequate glycaemic control despite optimal insulin therapy.

15. Dose of 400mg of sotagliflozin for the treatment of type 2 diabetes in patients with CKD3.

16. Dose of 400mg of sotagliflozin for the treatment to improve glycaemic control in patients with type 2 diabetes and CKD3.
